# EUROPEAN PATENT SPECIFICATION

(11) **EP 0 442 482 B1**
(45) Date of publication and mention of the grant of the patent: **01.06.1994**
(21) Application number: 91102042.8
(22) Date of filing: 13.02.1991
(51) Int. Cl.: A61B 17/068

(54) **Rotating head skin stapler**
Hautklammergerät mit rotierendem Kopf
Agrafeuse chirurgicale à tête rotative pour la peau

(30) Priority: 13.02.1990 US 479318
(43) Date of publication of application: 21.08.1991
(73) Proprietor: ETHICON INC., Somerville New Jersey 08876 (US)
(72) Inventor: Murray, Michael A., Bellevue, KY 41073 (US); Love, John F., Cincinnati, OH 45241 (US); Hughett, James D., Cincinnati, OH 45251 (US); Stephens, Randy R., Fairfield, OH 45014 (US); Schwemberger, Richard F., Cincinnati, OH 45247 (US)
(74) Representative: Strehl Schübel-Hopf Groening & Partner

(56) References cited:
- EP-A- 0 040 683
- EP-A- 0 094 752
- EP-A- 0 203 375
- EP-A- 0 229 453
- EP-A- 0 253 629
- EP-A- 0 337 874
- WO-A-87/04063
- GB-A- 2 019 296
- US-A- 4 043 504
- US-A- 4 202 480

## Description

The present invention is based on a surgical stapler having a base and a drive mechanism containing a trigger connected to said base and to a stapling mechanism. Said trigger undergoing a firing stroke to activate the stapling mechanism to fire staples, said stapling mechanism comprising ratchet means in contact with the trigger; said ratchet means maintaining said trigger at incremental position along said stroke; said trigger being rotationally connected to a pivot on the base and moving the stapling mechanism along a drive axis; a return spring maintaining a force on said trigger in the direction opposite that or said firing stroke; said ratchet means comprising a pawl in contact with said trigger and a rack attached to said base such that during this stroke said pawl contacts said rack in mating relationship with enough force to overcome the return spring force on said trigger and maintain said trigger along said stroke; said rack comprising a plurality of teeth; said pawl containing a stopping surface such that said stopping surface contacts said teeth and said teeth resist the return spring force on said trigger. A surgical stapler having the above-mentioned features is known from EP-A-40 683.

The invention has the object to avoid jamming of the staples.

The invention solves this problem by an improvement characterized in that said ratchet means being arranged along said drive axis; and said pawl comprising an engagement tab and said base comprising a block such that said tab engages said block at the beginning of said stroke and before said stopping surface engages said teeth.

These objects of the invention will be better understood by the following Detailed Description of the Drawings taken in conjunction with the Detailed Description of the Invention.

### Detailed Description of the Drawings

Fig. 1 is a perspective view of the rotating head skin stapler of this invention;
Fig. 2 is an exploded perspective view showing the replaceable staple cartridge removed from the rotating head;
Fig. 3 is a partial perspective view showing the rotating head in one possible orientation;
Fig. 4 is a partial perspective view of the rotating head in another possible orientation;
Fig. 5 is a cross-sectional view taken along line 5-5 of Fig. 2;
Fig. 6 is a view similar to Fig. 5 after compressing the handle and trigger;
Fig. 7 is a fragmentary view in cross-section of the trigger mechanism of the invention;
Fig. 8 is a fragmentary view of the buffer mechanism;
Fig. 9 is a plan view of the drive block and drive train rotating mechanism;
Fig. 10 is a perspective view of the drive block and drive train mechanism;

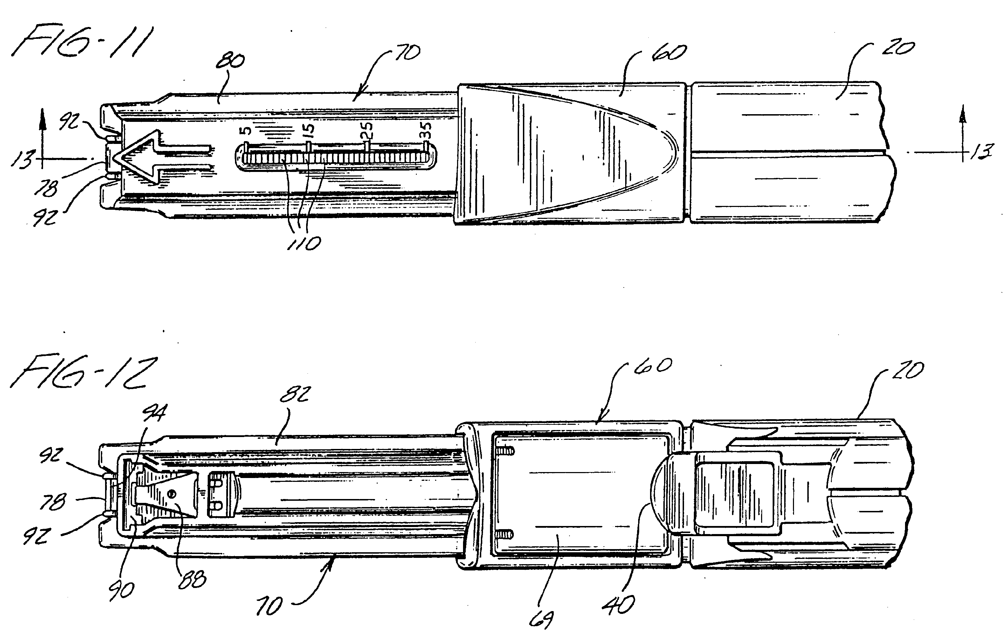

Fig. 11 is a partial top plan view of the rotating head skin stapler;
Fig. 12 is a partial bottom plan view of the rotating head skin stapler;

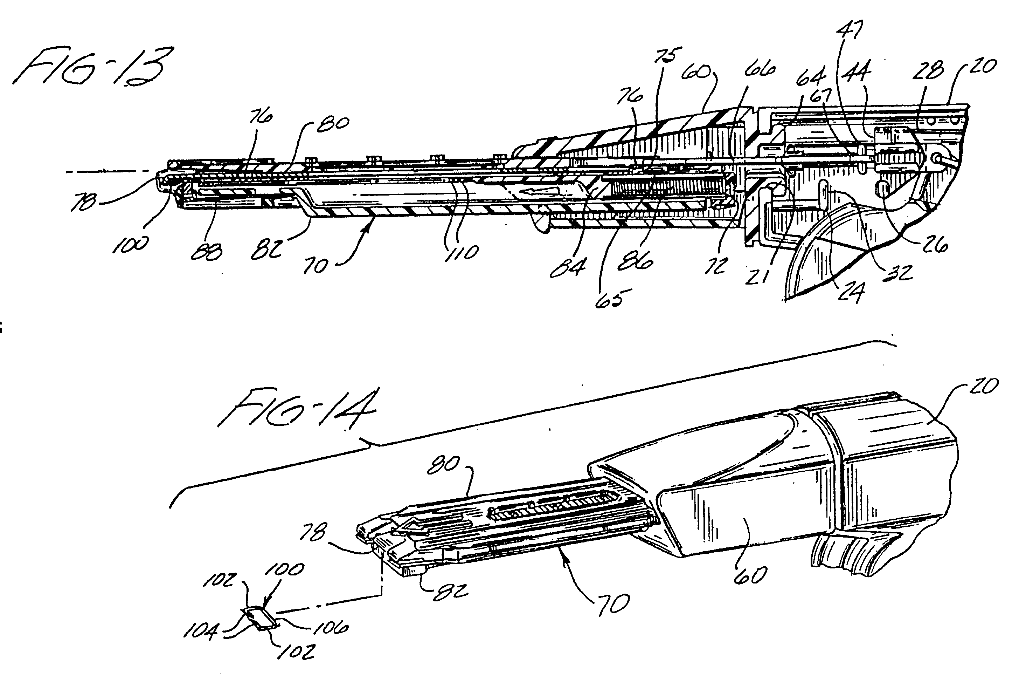

Fig. 13 is a cross-sectional view taken along line 13-13 of Fig. 11;
Fig. 14 is a partial perspective view showing a formed staple which released from the distal end of the staple cartridge;
Fig. 15 is a partial top plan view of the distal portion of the staple cartridge with the top of the cartridge partially broken away for clarity;
Fig. 16 is a cross-sectional view taken along line 16-16 of Fig. 15;
Fig. 17 is a partial bottom plan view of the staple cartridge just after forming the staple;
Fig. 18 is a cross-sectional view taken along line 18-18 of Fig. 17; and
Fig. 19 is an exploded perspective view of the staple cartridge of this invention.

### Detailed Description of the Invention

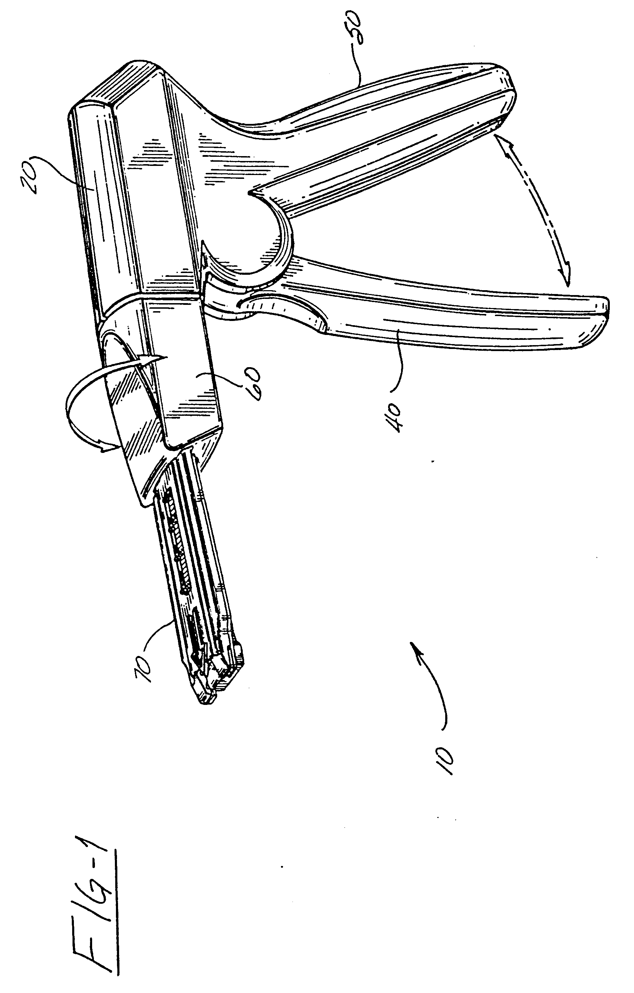

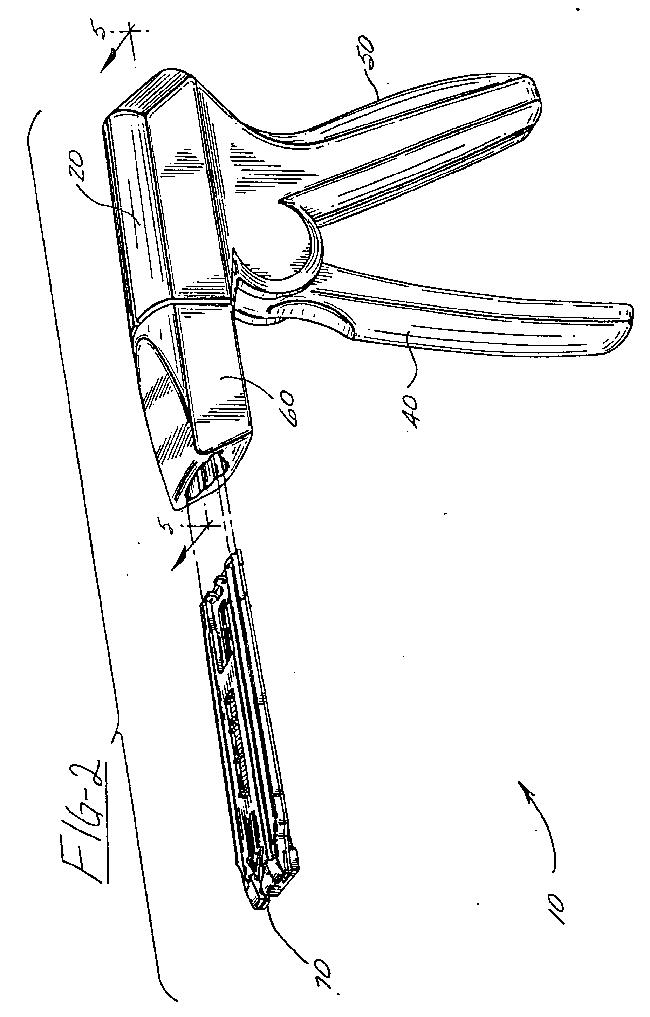

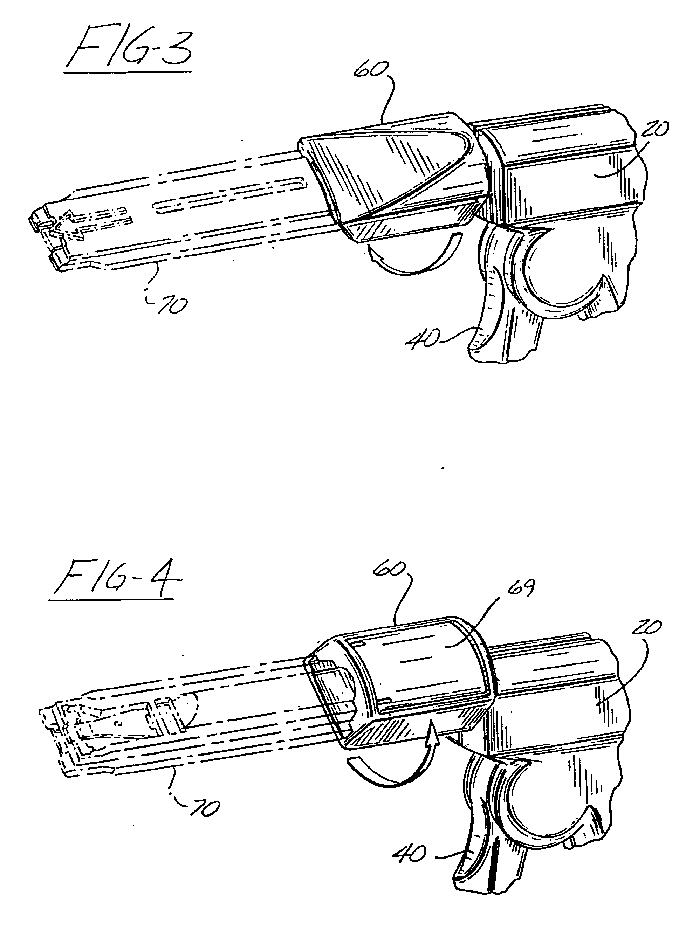

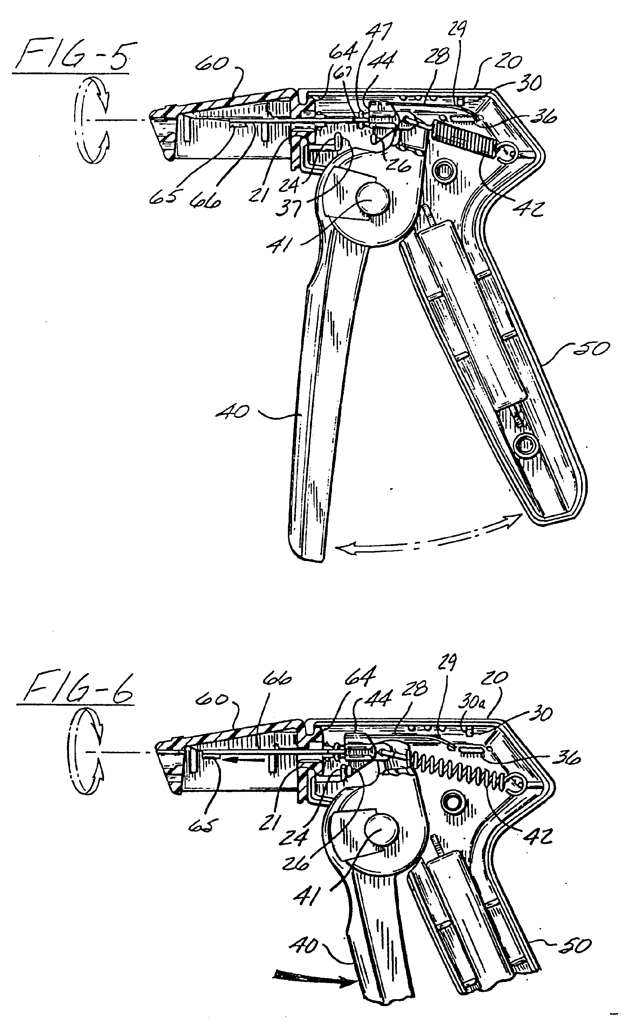

As seen in Figures 1 through 4, the rotating head skin stapler 10 contains a base 20 housing a drive mechanism which is activated by a trigger 40 squeezed within handle 50. The drive mechanism contained in the base 20 causes staples to be fired from cartridge 70. The rotating head 60 allows orientation of the cartridge 70 in any angular direction in relation to base 20 on the wound surface to be closed, as best seen in Figures 3 and 4.

Various aspects of the rotating head skin stapler 10 will now be explained. Intrinsic to the rotating head skin stapler 10 is rotating head 60. This rotating head 60 is attached to the base 20 by means of collar 64 enmeshed within holding cylinder 21 as better seen in Figures 5 and 6. Drive train 66 is rotatably connected at its proximal end to drive block 44 and at its distal end contacts former 76 within the plane of cartridge 70. Drive train 66 fits axially into collar 64 on rotating head 60. Drive train 66 is generally flat in shape, and constantly enmeshed between cartridge 70 and rotating head 60, as will be explained later. Because drive train 66 and cartridge 70 are fitted into the center of rotating head 60, and rotate about drive block 44, during rotation they maintain positional relationship with base 20, so that the orientation of cartridge 70 is optional to the user. For instance, as seen in Fig. 4, cartridge 70 has been rotated to expose head plate 69.

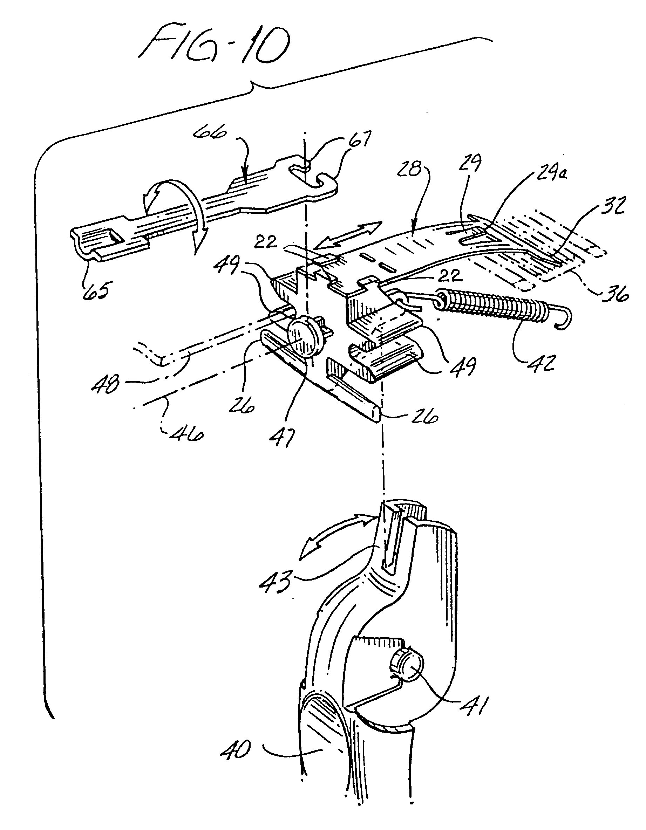

At its proximal end, drive train 66 is rotatably engaged with drive block 44. This is accomplished by inserting notched perforations 67 at the proximal end of drive train 66 around circular node 47 of drive block 44, as better seen in Figures 9 and 10. Thus, the notched perforations 67 are free to rotate about circular node 47 to allow drive train 66 to rotate about drive block 44, as seen in Figures 5 and 6. In this way, wherever rotating head 60 is oriented with drive train 66 contained therein, drive train 66 remains attached to drive block 44, and transfers force through the rotating head 60 and cartridge 70 combination.

For operation of the stapler, it is necessary for drive train 66 to have force imparted on it by drive link or drive block 44. As drive train 66 is rotatably attached by notched perforations 67 onto the drive block circular node 47, only force exerted along the same axis as drive train 66 will be imparted from drive block 44.

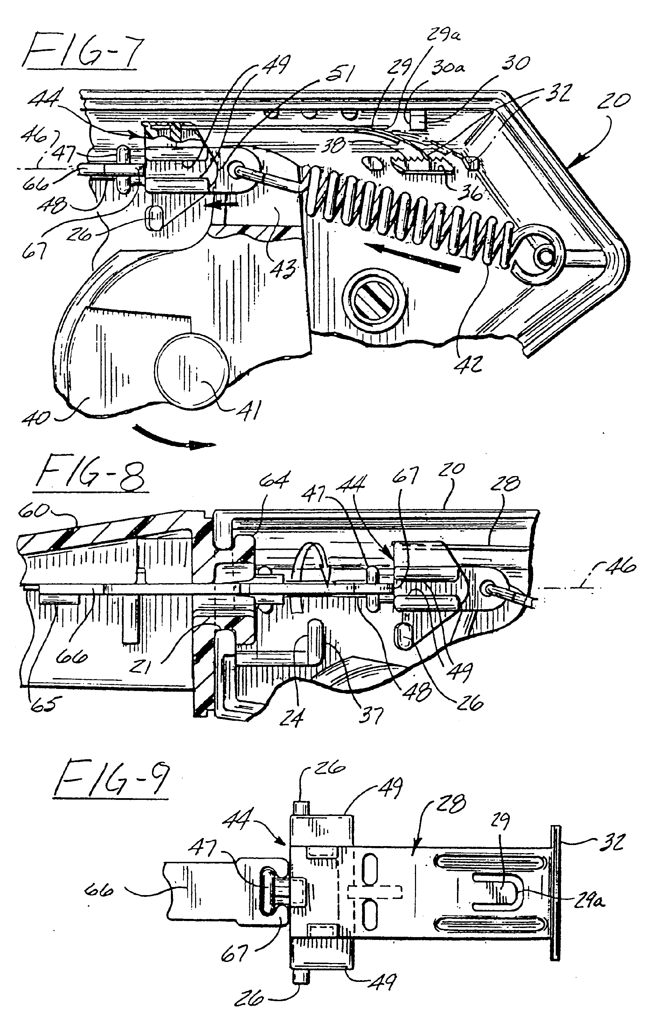

As better seen in the enlarged and various views of the interior of base 20 in Figures 5, 6, 7, 8, 9 and 10, drive block 44 is maintained on travel axis 46 through use of guide channels 48. When drive block 44 is moved linearly along guide channels 48, the wings 49 on drive block 44 are maintained about guide channels 48. Thus, drive block 44 is ensured of travel along travel axis 46. Trigger 40 contacts drive block 44, and therefore imparts force on drive block 44 along travel axis 46.

Trigger 40 rotates about pivot 41 within handle 50. As seen in Figure 7, elongated trigger projections 43 contact rear surface 51 of drive block 44. Rotation of the trigger 40 about pivot 41 necessarily causes the user to impart forces on drive block 44 along travel axis 46. This, in turn, causes motion of drive block 44 along travel axis 46, and operation of the stapling mechanism in cartridge 70.

Improvements to the rotating head skin stapler 10 are seen in the driving mechanism employed in use of trigger 40. Drive block 44 has attached to it a pawl 28, by means of tabs 22 folded over the pawl 28, as seen in Figures 9 and 10. When trigger 40 is cocked by rotation about pivot 41, drive block 44 causes pawl 28 to move linearly in unison with drive block 44 along axis 46. The rear of drive pawl 28 encounters surface 30a on engagement block 30 at surface 29a on engagement tab 29, as seen especially at Figures 5 and 7 in a position normal to travel axis 46. Engagement tab surface 29a becomes enmeshed with engagement block surface 30a to prevent rearward linear motion of drive block 44 along travel axis 46.

Relying further on Figures 6, 7,9 and 10, upon further motion of the trigger 40, the stopping surface 32 of drive pawl 28 contacts multi-tooth rack 36 on edges of teeth 38. Stopping surface 32 is normal to travel axis 46 and continues to prevent motion of drive block 44 into base 20 in incremental steps throughout the remainder of the stroke of trigger 40. Such continuous maintenance of the position of drive block 44 affirmatively prevents jamming of the stapler 10, by preventing drive block 44 and consequently trigger 40 from retracting linearly along travel axis 46 or recocking during a single stroke of trigger 40. The stapling mechanism in cartridge 70 will not reload, and therefore two staples will not be processed simultaneously at the forming site.

Accordingly, during motion of drive pawl 28 along multi-tooth rack 36, each of the teeth 38 hold drive pawl 28 at stopping surfaces 32 on edges of teeth until full rotation of the trigger 40 is accomplished. Then, the pawl 28 acts like a leaf spring and recoils so that the surface of pawl 28 clears the lower surface of multi-tooth rack 36. This allows drive spring pawl 28 to return to its original position. Return spring 42 causes trigger 40 to return drive block 44 along travel axis 46 after one full stroke of trigger 40.

As further seen in the enlarged view of drive block 44 as in Fig. 10, there are contained on block 44 winged-shaped buffers 26. These wing-shaped buffers 26 provide resistive force encountered by the user during the forward linear motion of drive block 44, near completion of the stroke of the trigger 40. Ordinarily, at the completion of a firing stroke, staples 100 have been formed, but the user continues to drive trigger 40. In order to reduce any "snap" in the feel of the trigger 40, due to the continued force of former 76 against staple crown 106 of the (now formed) staple 100, it is necessary to minimize forward linear motion of drive block 44 and spread the force over a larger surface area by imparting a resistive force opposite the direction of motion of drive block 44, and thus reduce the pressure exerted on drive block 44.

As seen in Figures 5, 6 and 8, block-shaped stops 24 are provided in base 20 which engage the buffers 26 near the end of the stroke of trigger 40. These stops 24 contain stopping surfaces 37 which cause the buffers 26 to elastically bend near the end of the stroke of trigger 40. In this way, the force actually imparted by the trigger 40 reduced by spreading forces over a larger surface area near the end of the stroke, and the user experiences no "snap" caused by impact of former 76 on staple 100 after complete staple forming.

In summary, the trigger anti-jamming mechanism and the drive link buffer of the invention accomplish the following steps: The multi-tooth rack 36 provides engagement surfaces on the teeth 38 which are normal to the travel axis 46 and therefore provide a resistive force parallel to the travel axis 46. The drive pawl 28, made of a resilient material to resist permanent deformation, engages the multi-tooth rack 36 in a direction normal to motion of drive block 44 to provide resistive forces parallel to travel axis 46. The engagement tab surface 29a on spring pawl 28 provides early engagement with block engagement surface 30a in order to prevent misfiring of the stapling mechanism in cartridge 70 at an earlier position of trigger 40 stroke. The engagement of the pawl 28 with the rack 36 allows the user to have a smoother feel of the surgical stapling instrument throughout the stroke of trigger 40.

In addition, with the buffers 26 molded as an integral part of the drive block 44, as the stroke of the former 76 approaches the final stage of contact between staple crown 106 and anvil forming surface 94 (as later explained), buffers 26 contact stopping surfaces 37. Because buffers 26 are elastic, they begin to bend and resist any continuing force imparted by drive block 44. In this way, forward motion of the block 44 is slowed and greatly reduces the impact of former 76 against the staple crown 106. This results in a more consistent force to form the staples, and avoids any snap felt by the user during trigger 40 stroke.

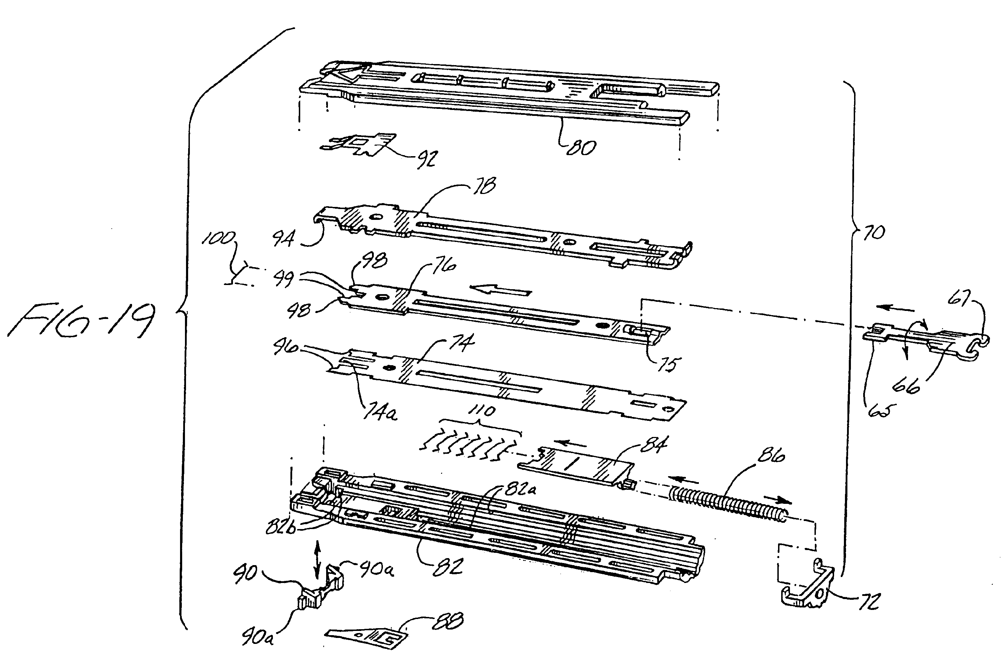

Other aspects of the invention are seen in the staple cartridge 70. Specifically, as seen in Figures 17 through 19 drive train 66 is connected to former 76 in the cartridge 70 by sliding plate 65 into gripping receiver 75. Former 76 contacts the first of a group of staples 100 at the head of staple stack 110. These staples 100 contain wings 102, legs 104, and crown 106. Lifter 90 holds a staple 100 in place and maintains staple 100 in position due to force imparted by spring 88 on lifter 90, as later explained. During formation of a staple 100, crown 106 contacts the forming surface 94 of anvil 78 at a forming site removed from staple stack 110. This is better explained in U.S. Patent No. 4,811,886, assigned to the common assignee as this invention, and incorporated herein by reference.

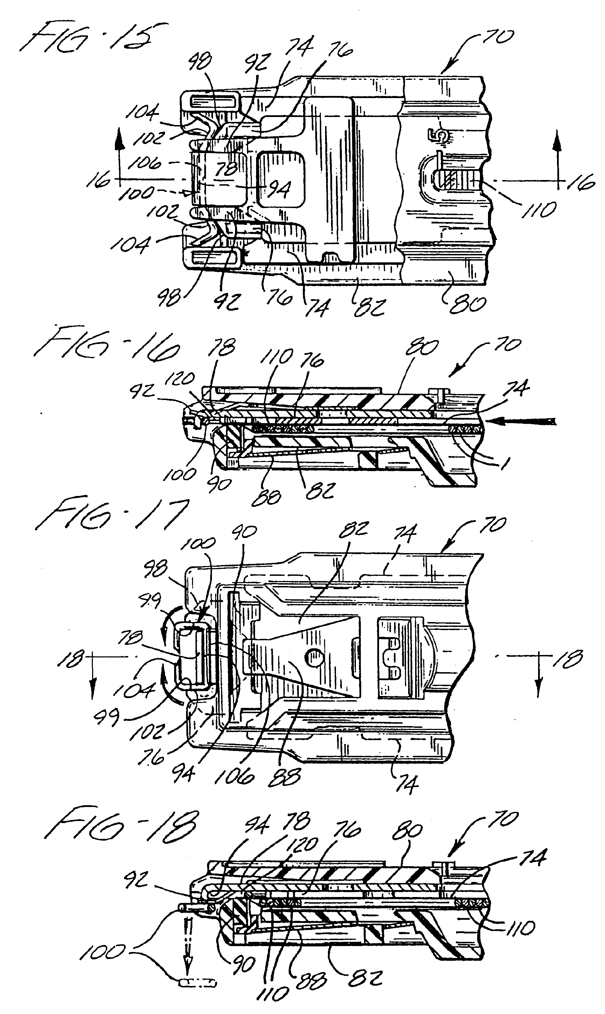

As seen in the views of the former 76 in Figures 15 and 19, former edges 98 are angled like the gull wing shaped wings 102 and legs 104 of staple 100. With the improved former edges 98, the legs 104 become self-centering within staple forming track 120, and force is kept on the inside edges of the staple legs 104 during forming. In so doing, the staple 100 stays centered on former 76 until staple 100 is formed around anvil forming surface 94. Alignment between former 76 and anvil 78 thus becomes the controlling alignment criterion, rather than relying on tolerances of staple 100. If the staple 100 is positioned slightly to one side of the anvil forming surface 94, the funnelling effect of the former edges 98 biases or "pulls" the staple 100 to the center, and controls it throughout forming of the staple 100. The continuous force imparted on the inside of staple legs 104 during the firing stroke decreases the possibility of malformation of the staple during forming, as seen in Figures 15 through 18.

In addition, as seen in Figures 15 and 17, anvil forming surface 94 on anvil 78 in the cartridge 70 is smaller in width than staple crown 106. In this way, crown 106 is shaped entirely around anvil forming surface 94. Because the crown 106 is wider than anvil forming surface 94, the cold worked areas of the staple found at the unions of crown 106 and wings 102 are shaped in spaced-apart relationship to the forming surface 94. Thus, the cold worked areas of the staple 100 are avoided during forming about forming surface 94, reducing forces necessary to form a staple 100.

Another improvement is seen in the lifter mechanism in cartridge 70 of the stapler. Lifter 90 is controlled by lifter spring 88 on the lower staple housing 82 of staple cartridge 70. Lifter spring 88 causes lifter 90 to move one staple 100 from the stack of staples 110 in staple feeding track 82a of the lower staple housing 82 of the cartridge 70. The staples in stack 110 are moved along feeding track 82a by feeder shoe 84, which is urged distally by feeder spring 86. Lifter spring 88 causes lifter 90 to lift a staple 100 across profile 96 in intermediate staple housing 74, which defines a vertical passage between parallel feeding track 82a and forming track 120.

The profile 96 on intermediate staple housing 74 has a shape corresponding to the staples 100 and maintains the staple 100 on lifter 90 properly within intermediate housing 74. Lifter 90 therefore prevents transfer and double loading of staples from the stack of staples in feeding track 82a onto the staple forming stack 120.

Retainer cap 72 holds together upper staple housing 80 and lower staple housing 82 and maintains feeder spring 86 in cartridge 70 so that the force urging staple stack 110 along feeder track 82a and into a staple forming track 120 is uninterrupted. Staple kick-off spring 92 causes the formed staples 100 to be kicked off from the anvil forming surface 94 when formed and placed in the skin and former 76 is retracted. Top staple housing 80 of cartridge 70 comprises the upper surface of forming track 120.

The staple transfer mechanism found in lifter 90 lifts the staple between the parallel staple stack 110 in staple feeding track 82a and staple forming track 120 incorporated in cartridge 70. The single staple 100 is supported along its crown 106 and legs 104 by the lifter 90 during lifting from the staple feeding track 82a to staple forming position in staple forming track 120. Gripping receiver 75 located on the distal end of the intermediate staple guide 74 provides resistive force to the motion of staple housing 90 and maintains the staple in contact with lifter 90 and profile 96 through motion between the staple feeding track 82a and staple forming track 120. Ears 90a on lifter 90 protrude transversely into channels 82b of lower staple housing 82 to guide motion of lifter 90. This staple transfer mechanism allows for reliable staple feeding in the staple cartridge 70 within a thin profile. This allows for improved visible staple placement onto the surgical site.

While the invention has been described in connection with a particularly preferred embodiment, it will be understood that the following claims are meant to describe the invention.

## Claims

1. Surgical stapler (10) having a base (20) and a drive mechanism (44) containing
a trigger (40) connected to said base (20) and to a stapling mechanism;
said trigger (40) undergoing a firing stroke to activate the stapling mechanism to fire staples (100),
said stapling mechanism comprising ratchet means (28, 36) in contact with said trigger (40);
said ratchet means (28, 36) maintaining said trigger (40) at incremental positions along said stroke;
said trigger (40) being rotationally connected to a pivot (41) on said base (20) and moving said stapling mechanism along a drive axis (46);
a return spring (42) maintaining a force on said trigger (40) in a direction opposite that of said firing stroke; said ratchet means (28, 36) comprising a pawl (28) in contact with said trigger (40) and a rack (36) attached to said base (20) such that during the stroke said pawl (28) contacts said rack (36) in mating relationship with enough force to overcome the return spring force (42) on said trigger (40) and maintain said trigger along its stroke;
said rack (36) containing a plurality of teeth (38); and
said pawl (28) containing a stopping surface (32) such that said stopping surface (32) contacts said teeth (38) and said teeth resist the return spring force (42) on said trigger (40);
characterized by
said ratchet means (28, 36) being arranged along said drive axis (46); and
said pawl (28) comprising an engagement tab (29) and said base (20) comprising a block (30) such that said tab (29) engages said block (30) at the beginning of said stroke and before said stopping surface (32) engages said teeth (38).

2. Surgical stapler of claim 1 having stapling means manually activated by the stroke of a trigger (40) in a base (20) for firing staples (100), characterized by opposing means in said base (20), said opposing means (24, 26) acting on said trigger (40) at the end of said stroke such that said opposing means (24, 26) act on said trigger (40) during formation of said staple (100).

3. Surgical stapler of claim 1 having a plurality of staples (100) positioned in a staple stack (110) and a staples forming mechanism (76) positioned away from said staple stack (110) characterized by lifter means (90) for taking the first staple (100) from said staple stack (110), and transferring said first staple (100) to said forming mechanism (76) while continually maintaining orientation of said staple (100) during transfer.

4. Surgical stapler of claim 1 or 3 having a staple former (76) which shapes surgical staples (100) about an anvil (78), said former (76) having a rectangular inner profile (96) and edges (98) for contacting said staple legs (104), said unformed staple (100) having a crown (106) and a pair of legs (104) angularly connected to said crown (106), said staple legs (104) bending at the union with said crown (106) along the inner edges (98) of said former (76) and about said anvil (78), characterized by said edges (98) contacting said legs (104) before said inner profile begins to bend said legs (104).

5. Surgical stapler of claim 1, 3 or 4 containing a plurality of unformed staples (100), each said staple (100) having a crown (106) and a pair of legs (104), said staple (100) formed by bending said staple (100) using a former (76) to bend said legs (104) about an anvil (78), characterized by said unformed crown (106) being longer than said anvil (78).

## Patentansprüche

1. Chirurgisches Klammergerät (10) mit einem Gehäuse (20) und einem Antriebsmechanismus (44), aufweisend
eine Auslöseeinrichtung (40), die mit dem Gehäuse (20) und einem Klammermechanismus verbunden ist;
wobei die Auslöseeinrichtung (40) einen Abschußhub erfährt, um den Klammermechanismus zum Abschießen von Klammern (100) zu aktivieren,
wobei der Klammermechanismus eine Sperrklinkeneinrichtung (28, 36) in Kontakt mit der Auslöseeinrichtung (40) aufweist;
wobei die Sperrklinkeneinrichtung (28, 36) die Auslöseeinrichtung an inkrementalen Positionen längs des Hubs hält;
die Auslöseeinrichtung (40) drehbar mit einem Drehzapfen (41) auf dem Gehäuse (20) verbunden ist und den Klammermechanismus längs einer Antriebsachse (46) bewegt;
eine Rückholfeder (42) eine Kraft auf die Auslöseeinrichtung (40) in einer Richtung entgegengesetzt der des Abschußhubs aufrechterhält;
die Sperrklinkeneinrichtung (28, 36) eine Sperrklinke (28) aufweist, die sich in Kontakt mit der Auslöseeinrichtung (40) befindet, und eine Zahnstange (36), die am Gehäuse (20) befestigt ist, derart, daß während des Hubs die Sperrklinke (28) sich mit der Zahnstange (36) in Eingriffskontakt befindet, mit einer ausreichenden Kraft, um die Kraft der Rückholfeder (42) auf die Auslöseeinrichtung (40) zu überwinden und die Auslöseeinrichtung längs ihres Hubs zu halten;
wobei die Zahnstange (36) eine Vielzahl von Zähnen (38) aufweist; und
die Sperrklinke (28) eine Anschlagfläche (32) aufweist, derart, daß die Anschlagfläche (32) in Kontakt mit den Zähnen (38) ist, und die Zähne der auf die Auslöseeinrichtung (40) wirkenden Kraft entgegenwirken;
**dadurch gekennzeichnet, daß**
die Sperrklinkeneinrichtung (28, 36) längs der Antriebsachse (46) angeordnet ist, und daß die Sperrklinke (28) einen Eingriffsdorn (29) aufweist und das Gehäuse (20) einen Block (30) aufweist, derart, daß der Dorn (29) den Block (30) am Beginn des Hubs und bevor die Anschlagfläche (32) mit den Zähnen (38) in Eingriff kommt, erfaßt.

2. Chirurgisches Klammergerät nach Anspruch 1 mit einer Klammereinrichtung, die manuell durch den Hub einer Auslöseeinrichtung (40) in einem Gehäuse (20) zum Abschuß von Klammern (100) betätigt wird, **gekennzeichnet durch** Gegenmittel in dem Gehäuse (20), wobei die Gegenmittel (24, 26) auf die Auslöseeinrichtung (40) am Ende des Hubs wirken, derart, daß die Gegenmittel (24, 26) während der Ausbildung der Klammer (100) auf die Auslöseeinrichtung (40) wirken.

3. Chirurgisches Klammergerät nach Anspruch 1 mit einer Vielzahl von Klammern (100), die in einem Klammerstapel (110) positioniert sind, und einem Klammerformungsmechanismus (76), der vom Klammerstapel (110) entfernt positioniert ist, **gekennzeichnet durch** eine Anhebeeinrichtung (90) zur Entnahme der ersten Klammer (100) vom Klammerstapel (110) und Überführung der ersten Klammer (100) zum Formungsmechanismus (76), wobei die Orientierung der Klammer (100) während der Überführung kontinuierlich aufrechterhalten wird.

4. Chirurgisches Klammergerät nach Anspruch 1 oder 3 mit einem Klammerformwerkzeug (76), das chirurgische Klammern (100) um einen Amboß (78) herum formt, wobei das Formwerkzeug (76) ein rechteckförmiges inneres Profil (96) und Kanten (98) zur Kontaktierung der Klammerschenkel (104) aufweist, wobei die ungeformte Klammer (100) eine Krone (106) und ein Paar Schenkel (104) aufweist, die abgewinkelt mit der Krone (106) verbunden sind, wobei die Klammerschenkel (104) im Übergang zur Krone (106) längs den Innenkanten (98) des Formwerkzeugs (76) und um den Amboß (78) herum gebogen werden, **dadurch** **gekennzeichnet, daß**
die Kanten (98) die Schenkel (104) berühren, bevor das innere Profil beginnt, die Schenkel (104) zu verbiegen.

5. Chirurgisches Klammergerät nach Anspruch 1, 3 oder 4 mit einer Vielzahl von ungeformten Klammern (100), wobei jede Klammer (100) eine Krone (106) und ein Paar Schenkel (104) aufweist, wobei die Klammer (100) durch Biegen der Klammer (100) unter Verwendung eines Formwerkzeuges gebildet wird, um die Schenkel (104) um einen Amboß (78) herum zu biegen,
**dadurch gekennzeichnet, daß**
die unverformte Krone (106) länger ist als der Amboß (78).

## Revendications

1. Agrafeuse chirurgicale (10) comportant une base (20) et un mécanisme d'entraînement (44) comportant
une gâchette (40) reliée à ladite base (20) et au mécanisme d'agrafage;
ladite gâchette (40) subissant une course de mise en oeuvre pour actionner le mécanisme d'agrafage pour mettre en oeuvre des agrafes (100)
ledit mécanisme d'agrafage comportant des moyens formant rochet (28, 36) en contact avec ladite gâchette (40);
lesdits moyens formant rochet (28, 36) maintenant ladite gâchette (40) au niveau de positions incrémentielles le long de ladite course;
ladite gâchette (40) étant reliée de manière rotative à un pivot (41) situé sur ladite base (20) et déplaçant ledit mécanisme d'agrafage le long d'un axe d'entraînement (46);
un ressort de rappel (42) maintenant sur ladite gâchette (40) une force de sens opposé à celui de ladite course de mise en oeuvre; lesdits moyens formant rochet (28, 36) comportant un cliquet (28) en contact avec ladite gâchette (40) et une crémaillère (36) fixée sur ladite base (20) de sorte que pendant la course ledit cliquet (28) soit en contact avec ladite crémaillère (36) en étant relié avec une force suffisante pour surmonter la force exercée par le ressort de rappel (41) sur ladite gâchette (40) et maintenir ladite gâchette le long de sa course;
ladite crémaillère (36) comportant plusieurs dents (38), et
ledit cliquet (28) comportant une surface d'arrêt (32) telle que ladite surface d'arrêt (32) soit en contact avec lesdites dents (38) et que lesdites dents résistent à la force du ressort de rappel (2) agissant sur ladite gâchette (40);
caractérisée en ce que
lesdits moyens formant rochet (28, 36) sont agencés le long dudit axe d'entraînement (46); et
ledit cliquet (28) comporte une patte de contact (29) et ladite base (20) comporte une butée (30) telle que ladite patte (29) soit en contact avec ladite butée (30) au moment du début de ladite course et avant que ladite surface d'arrêt (32) ne soit en contact avec lesdites dents (38).

2. Agrafeuse chirurgicale selon la revendication 1 comportant des moyens d'agrafage actionnés de manière manuelle par la course d'une gâchette (40) dans une base (20) pour mettre en oeuvre des agrafes (100), caractérisée par des moyens d'opposition situés dans ladite base (20), lesdits moyens d'opposition (24, 26) agissant sur ladite gâchette (40) à la fin de ladite course de sorte que lesdits moyens d'opposition (24, 26) agissent sur ladite gâchette (40) pendant la mise en forme de ladite agrafe (100).

3. Agrafeuse chirurgicale selon la revendication 1 comportant un ensemble d'agrafes (100) positionnées en un empilage d'agrafes (110) et un mécanisme de mise en forme (76) d'agrafes positionné en dehors dudit empilage d'agrafes (100), caractérisée par des moyens formant dispositif de levage (90) destinés à prendre la première agrafe (100) dudit empilage d'agrafes (110), et à transférer ladite première agrafe (100) audit mécanisme de mise en forme (76) tout en maintenant de manière continue l'orientation de ladite agrafe (100) pendant le transfert.

4. Agrafeuse chirurgicale selon la revendication 1 ou 3 comportant un dispositif de mise en forme (76) d'agrafe qui met en forme les agrafes chirurgicales (100) autour d'une enclume (78), ledit dispositif de mise en forme (76) ayant un profil intérieur (96) rectangulaire et des bords (98) destinés à être en contact avec lesdites pattes (104) d'agrafe, ladite agrafe (100) non mise en forme ayant une couronne (106) et une paire de pattes (104) reliées de manière anguleuse à ladite couronne (106), lesdites pattes (104) d'agrafe se pliant au niveau de la liaison avec ladite couronne (106) le long des bords intérieurs (98) dudit dispositif de mise en forme (76) et autour de ladite enclume (78), caractérisée en ce que lesdits bords (98) sont en contact avec lesdites pattes (104) avant que ledit profil intérieur ne commence a plier lesdites pattes (104).

5. Agrafeuse chirurgicale selon la revendication 1, 3 ou 4 comportant un ensemble d'agrafes (100) non mises en forme, chaque dite agrafe (100) comportant une couronne (106) et une paire de pattes (104), ladite agrafe (100) étant mise en forme par pliage de ladite agrafe (100) par utilisation d'un dispositif de mise en forme (76) destiné à plier lesdites pattes (104) autour d'une enclume (78), caractérisée en ce que ladite couronne (106) non mise en forme est plus grande que ladite enclume (78).
